# EUROPEAN PATENT APPLICATION

(11) **EP 3 461 416 A1**
(43) Date of publication of application: **03.04.2019**
(21) Application number: 17193752.7
(22) Date of filing: 28.09.2017
(51) Int. Cl.: A61B 6/12, A61B 6/00, A61B 8/12

(54) **GUIDING AN INTRAVASCULAR US CATHETER**

(71) Applicant: Koninklijke Philips N.V., 5656 AE Eindhoven (NL)
(72) Inventor: HAASE, Christian, 5656 AE Eindhoven (NL); SCHMITT, Holger, 5656 AE Eindhoven (NL); PREVRHAL, Sven, 5656 AE Eindhoven (NL)
(74) Representative: de Haan, Poul Erik

(57) **Abstract**

The invention relates to a system and a method for guiding an intravascular ultrasound catheter device comprising an ultrasound probe to a potential lesion in a vascular tree (31). In the system, position information of the probe are provided for displaying to a user and/or for an automated processing of the position information. An evaluation unit is configured to receive a diagnostic image of the vascular tree, to determine values of at least one vessel parameter for a plurality of locations in the vascular tree, and to identify at least one location associated with an abnormal value of the at least one vessel parameter. Further, a mapping unit is configured to provide a visual indication (32) of the at least one location in the visualization of the vascular tree and/or to provide information about the at least one location for use in the automated processing of the position information.

## Description

### FIELD OF THE INVENTION

The invention generally relates to a guidance of an intravascular ultrasound (IVUS) catheter. More specifically, the invention relates to a system and a method for guiding an IVUS catheter comprising an ultrasound (US) probe to a potential lesion.

### BACKGROUND OF THE INVENTION

IVUS uses a miniaturized US probe attached to a catheter device, which is introduced into the vascular tree of a patient in order to acquire in-situ US images of the blood vessels of the vascular tree. Since the US waves penetrate into the wall of the blood vessels, insight can be gained into the condition of these walls. In clinical practice, IVUS is applied to examine lesions in the vascular tree, particularly in the coronary arteries, which may particularly be caused by a buildup of plaque within the walls of blood vessels. The accumulation of such plaque results in lesions that may cause heart attack and stenosis. IVUS is particularly useful to determine the amount of plaque buildup and the plaque composition as wells as the degree of stenosis. This helps in assessing the severity of the lesions and in therapy planning such as the sizing and positioning of a stent.

In order to guide an IVUS examination, x-ray fluoroscopy is conventionally used. Thus, live x-ray images of the vascular system are acquired that show the IVUS catheter device as it is navigated through the vascular tree. In order to improve the visibility of the blood vessels in the x-ray images, a contrast agent may additionally be injected to the vascular tree. Using the resulting images, the operator can track the position of the IVUS catheter device and the included US probe and steer the IVUS catheter on the basis of the position information.

In this procedure, regions of the vascular tree may be imaged by means of the US probe while the US probe is being moved through the blood vessels of the vascular tree. In this process, the US catheter device may be steered manually and may specifically be navigated to locations or smaller regions of the vascular tree comprising potential lesions. Such potential lesions may be identified on the basis of the x-ray fluoroscopy image. However, it is sometimes difficult to identify potential lesions in the x-ray fluoroscopy image so that there is a certain risk that potential lesions are not examined in the IVUS examination. This is particularly the case for vessels with outward remodeling. These vessels may have a high plaque burden but may show no visible narrowing of the vessel in the fluoroscopy images. Due to the high plaque volume these vessels may nevertheless have a high risk of plaque rupture and therefore pose a high risk to cause an acute myocardial event.

Moreover, it is also possible to acquire images of greater sections of the blood vessels of the vascular tree e.g. in a so-called pullback procedure. In such a procedure, the operator may navigate the US probe to a certain position in the vascular tree. Starting from this position, the IVUS catheter device is then pulled back and during this movement of the IVUS catheter device US images are acquired. During the pullback procedure, the IVUS catheter device may be steered manually or automatically. In both cases, the IVUS catheter device is usually pulled back with certain (approximately) fixed velocity. However, in some cases, this velocity may be too high for imaging lesions located along the pullback path with a depth of detail sufficient for a careful examination of such lesions.

### SUMMARY OF THE INVENTION

In view of the above, it is an object of the present invention to improve IVUS examinations of a vascular system such that potential lesions can be located and examined more reliably and detailed.

In accordance with one aspect, the invention provides a system for guiding an IVUS catheter device comprising a US probe to a potential lesion in a vascular tree. The system comprises a localization unit configured to provide position information of the US probe for displaying to a user in a visualization of the vascular tree and/or for an automated processing of the position information. Further, the system comprises an evaluation unit configured to receive a diagnostic image of the vascular tree, to determine values of at least one vessel parameter for a plurality of locations in the vascular tree on the basis of the diagnostic image, and to identify at least one location in the vascular tree associated with an abnormal value of the at least one vessel parameter. Moreover, the system comprises a mapping unit configured to provide a visual indication of the at least one location in the visualization of the vascular tree and/or to provide information about the at least one location for use in the automated processing of the position information.

In the provided system, locations associated with an abnormal value of at least one vessel parameter may correspond to potential lesions. Advantageously, these locations are identified on the basis of an evaluation of a diagnostic image. This allows for a more reliable identification of potential lesions in the vascular system of a patient. The relevant locations are then marked in the visualization of the vascular tree by means of visual indications so that the operator of the imaging system can navigate the US probe to these locations in order to acquire US images for examining these locations. In addition or as an alternative, information about the locations is provided for use in the automated processing of the position information. This automated processing may particularly comprise an automated steering of the IVUS catheter device.

The diagnostic image may be a three-dimensional image of the vascular tree. In particular, the diagnostic image may be a computed tomography (CT) image, and the evaluation of the CT image in the evaluation unit may correspond to a computed tomography angiography (CTA) evaluation. However, the diagnostic image may likewise be acquired using another imaging modality, such as for example, magnetic resonance (MR) imaging. Moreover, the invention is not limited to three-dimensional diagnostic images. Rather, the diagnostic image may also be a two-dimensional image.

Abnormal values of the at least one parameter that may be indicative of potential lesions may particularly correspond to unusually high or low values of the at least one parameter. Therefore, the evaluation unit may be configured to identify an abnormal value of the at least one parameter if the value of the parameter is smaller than a predetermined lower threshold or greater than a predetermined upper threshold.

The at least one vessel parameter may comprises at least one of a wall shear stress acting on an inner wall of vessels of the vascular tree, a wall thickness of the walls of vessels of the vascular tree and a parameter indicating a calcification of the vessel wall. It has been found that abnormal values of these parameters are indicative of potential lesions, particularly of lesions that often cannot be detected in x-ray fluoroscopy images. Therefore, these parameters are especially suitable for identifying potential lesions in the vascular tree that may be further examined using IVUS. The wall shear stress may be determined on the basis of a simulation of a blood flow through the vascular tree performed based on the geometry of the vascular tree that is determined using the diagnostic image thereof, which may particularly be a three-dimensional image in this embodiment. The wall thickness may directly be derived from the vessel geometry as shown in the diagnostic image and also a calcification of the vessel wall may be visible in the diagnostic image, particularly in case a CT image is used.

In one embodiment of the invention, the localization unit is configured to provide the position information of the US probe with respect to a tracking frame and the evaluation unit is configured to identify the at least one location in an imaging frame of the diagnostic image. Therefore, the mapping unit may be configured to transform the at least one location into the tracking frame to provide the visual indication of the least one location in the visualization of the vascular tree and/or to provide the information about the at least one location for use in the automatic processing of the position information. In this context, the term "frame" particularly denotes a frame of a reference having a certain position and orientation in space. In each frame, a certain position may particularly be indicated by means of coordinates or other parameters relating to the respective frame.

In a further embodiment of the invention, the localization unit is configured to acquire live images of the vascular tree and the live images may correspond to the visualization of the vascular tree. In this embodiment, the aforementioned tracking frame may correspond to an imaging frame associated with the live images. In a related embodiment, the mapping unit is configured to provide a visual indication of the at least one location in the live images. The live images may particularly be x-ray fluoroscopy images. However, the live images may likewise be acquired using another imaging modality.

Moreover, one embodiment of the invention comprises that the evaluation unit is configured to segment the vascular tree in the diagnostic image to obtain a segmented vascular tree and to determine the values of the at least one vessel parameter on the basis of the segmented vascular tree. The segmented vascular tree particularly shows the geometry of the vascular tree and on the basis of this geometry, the values of the at least one vessel parameter may be estimated. In case of the vessel parameters referred to above, the wall thickness of the vessels included in the segmented vascular tree may particularly be determined based on the geometry of the vascular system as represented by the segmented vascular tree and/or the blood flow in the vascular tree may be simulated on the basis of this geometry in order to determine the wall shear stress acting on the vessel walls. In order to carry out this evaluation, a three-dimensional diagnostic image may particularly be used.

When the location unit acquires live images of the vascular tree, the mapping unit may be configured to provide a visual indication of the at least one location associated with abnormal values of the at least one vessel parameter in these images by overlaying the images with one or more marker(s) highlighting the at least one location. In a further embodiment, the mapping unit is configured to annotate the segmented vascular tree with an indication of the at least one location to obtain an annotated segmented vascular tree and to overlay the live images with the annotated segmented vascular tree. By means of such an overlay, it is not only possible to highlight the locations associated with abnormal parameter values but also the visibility of the vascular tree in the live images can be improved by means of the overlaid annotated version of the segmented vascular tree.

In one embodiment of the invention, the localization unit is configured to automatically track the position of the US probe to provide position information for the automated processing. The automatic tracking of the US probe may likewise be carried out on the basis of the live images. Likewise, a non image-based tracking technique may be applied, such as, for example, electromagnetic (EM) tracking, impedance tracking, optical shape sensing and satellite-based tracking. Moreover, in case of a pullback procedure, the US probe may be automatically tracked along the pullback path on the basis of the distance covered by the US probe during the pullback procedure, where the distance may be calculated based on the velocity of the US probe during the pullback procedure. Further, the US probe may be tracked along its path through the vascular tree on the basis of the US images acquired by means of the US probe and on the basis of the diagnostic image. In the US images, certain landmark features, such as a plaque volume, may be identified and the same feature may be identified in the diagnostic image in order to determine the position of the US probe in the vascular tree when it acquires an image including a landmark feature.

In one embodiment of the invention, the system further comprises a steering unit configured to automatically steer the intravascular ultrasound catheter device and the automated processing of the position information comprises an automated steering of the intravascular ultrasound catheter device based on the position information by means of the steering unit. In this embodiment, the information about the at least one location can be used in the automatic steering of the catheter device. In one related embodiment, the steering unit may automatically steer the US probe to the at least one location. In a further related embodiment, the steering unit is particularly configured to steer the IVUS catheter device in an automated pullback procedure on the basis of information about the at least on location associated with the abnormal value of the vessel parameter. In particular, the steering unit may be configured to compare a position of the US probe during the automated pullback procedure with the at least one location and to control a velocity of the US probe during the automated pullback procedure on the basis of the result of the comparison.

In one implementation, the steering unit is configured to move the US probe with a first velocity when the distance between its position and the at least one location is greater than a predefined distance and to move the US probe with a second velocity when the distance between its position and the at least one location is greater than the predefined distance, the second velocity being lower than the first velocity. Thus, the US probe passes the at least one location with a reduced velocity that may allow for imaging the at least one location with a greater depth of detail. In particular, this may allow for imaging the at least one location with an increased spatial resolution along the pullback direction, when the frame rate is kept at a fixed value during the pullback procedure.

In a further embodiment, the automated processing of the position information comprises an adaption of at least one image acquisition parameter of the US probe and/or to select a predetermined image evaluation procedure for evaluating an image acquired by the ultrasound probe on the basis of a distance between a position of the US probe and the at least one location.

The at least one image acquisition parameter may be adapted when the distance between the determined position of the US probe and the at least one location is smaller than a predetermined threshold value. This allows for acquiring ultrasound images of the at least one location using a modified image acquisition parameter, which differs from an image acquisition parameter used for imaging other regions of the vascular tree. Hereby, the image acquisition parameters may be optimized to image the potential lesion at the at least one location. Examples of related image acquisition parameters include the US frequency used for imaging and/or the frame rate used by the US probe to acquire images. In a further example, the US probe may be operable in an imaging mode and in a flow meter mode for measuring and visualizing blood flow, and the US probe may be controlled to switch to the flow meter mode at the at least one location. The predetermined image evaluation procedure may be selected for evaluating images that are acquired when the distance between the position of the US probe and the at least one location is smaller than a predetermined threshold. Hereby, it is possible to apply special image evaluation procedures for evaluating images of the potential lesion at this location.

In accordance with a further aspect, the invention provides method for guiding an IVUS catheter device comprising an US probe to a potential lesion in a vascular tree. The method comprises the following steps: (i) providing position information of the US probe for displaying to a user in a visualization of the vascular tree and/or for an automated processing of the position information; (ii) receiving a diagnostic image of the vascular tree; (iii) determining values of at least one vessel parameter for a plurality of locations in the vascular tree on the basis of the diagnostic image; (iv) identifying at least one location in the vascular tree associated with an abnormal value of the vessel parameter; and (v) providing a visual indication of the at least one location in the visualization of the vascular tree and/or providing information about the at least location for use in the automated processing of the position information.

In accordance with a further aspect, the invention provides a computer program comprising program code for causing a computer device to carry out the method, when the computer program is executed in the computer device.

It shall be understood that the system of claim 1, the method of claim 14 and the computer program of claim 15 have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

In the following drawings:
Fig. 1 schematically and exemplarily shows components of an IVUS imaging system according to one embodiment of the invention,
Fig. 2 schematically and exemplarily shows a live image of a vascular tree overlaid by an annotated segmented version of the vascular tree highlighting locations associated with abnormal values of a vessel parameter, and
Fig. 3 schematically and exemplarily shows an annotated segmented vascular tree in a further annotated version.

### DETAILED DESCRIPTION OF EMBODIMENTS

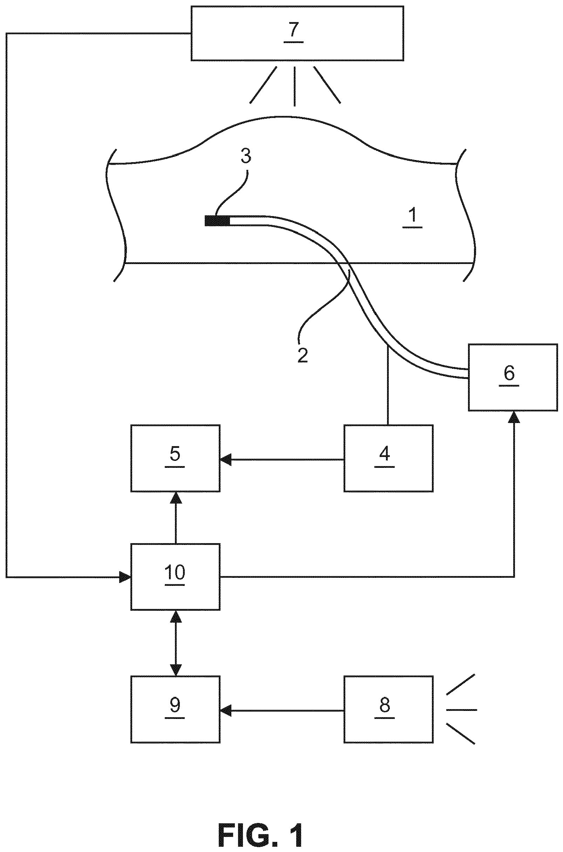

Fig. 1 schematically and exemplarily shows components of an IVUS imaging system according to an embodiment. The IVUS imaging system comprises an intravascular catheter device 2 comprising an US probe 3 for acquiring US data. The catheter device 2 is electronically coupled to an US unit 4, which receives the US data and generates US images on the basis thereof. The US images can be displayed to an operator of the imaging system at a display device 5, which is electronically coupled to the US unit 4 and which may be configured as a monitor device, for example.

The intravascular catheter device 2 has an elongated body with a distal end for percutaneous introduction into a vascular system of a patient 1. When the catheter device 2 is introduced in the patient's vascular system, it is steered from outside the patient body 1 by moving the proximal end section of the catheter device 2. This may be done manually by the operator of the imaging system and/or automatically by means of a steering unit 6, which may be mechanically coupled to the proximal end section of the catheter device 2. The steering unit 6 may particularly be used to perform an automated motorized pullback of the catheter device 2. In this procedure, the catheter device 2 may be manually navigated through the patient's vascular system to a certain position. Than the catheter device 2 may be automatically pulled backwards through the vessels by the steering unit 6 and during the backwards motion of the catheter device 2, US images may be acquired.

The US probe 3 is arranged at the distal end section of the intravascular catheter device 2. The US probe 3 may include a single miniaturized transducer element or an arrangement of several miniaturized transducer elements. The US probe 3 is configured to emit US signals and to sense echoes of the emitted US signals in a way known to the person skilled in the art as such, where the sensed echo signals are transmitted to the US unit 4 for the generation of the US images. The transducer element(s) may be configured as piezoelectric micro-machined US transducer(s) (PMUT(s)) fabricated on a microelectromechanical system (MEMS) substrate using a polymer piezoelectric material, for example as disclosed in US 6,641,540. In alternative embodiments, the transducer element(s) is/are configured as piezoelectric PZT (lead circonate titanate) transducer(s), capacitive micro-machined ultrasound transducer(s) (CMUT(s)) or in accordance with another suitable technology for manufacturing ultrasound transmitter and receiver arrangement(s).

Further, the imaging system comprises a tracking unit 7, which provides for a manual and/or automatic tracking of the catheter device 2 and particularly the US probe 3 included therein during operation thereof, i.e. within the vascular system of a patient 1. The tracking unit 7 particularly allows for tracking the position of the US probe 3 with respect to a certain fixed tracking frame, which corresponds to a frame of reference having defined position and orientation with respect to the patient body 1 during the examination of the patient 1. Particularly in order to allow for a manual tracking of the catheter device 2, the tracking unit 7 provides a visualization of the vascular system of the patient 1 including the catheter device 2 and/or an indication of its position. The visualization may be output at the display unit 5 so that it can be viewed by the operator and used when steering the catheter device 2.

In one embodiment, the US probe 3 is tracked using an image-based tracking technique. In this embodiment, the tracking unit 7 is configured to acquire two-dimensional or three-dimensional live tracking images of the inner of the patient body 1 including the area of the vascular system in which the US transducer 3 is operated. For this purpose, the tracking unit 7 applies a suitable imaging modality, which allows for acquiring images showing the patient's vascular system and the catheter device 2 moving therein. In this embodiment, the acquired live images can be directly used as the aforementioned visualization of the vascular system, which may be output at the display unit 5. Further, the live images include an imaged plane (in case of two-dimensional images) or volume (in case of three-dimensional images) which defines the tracking frame, where the position and orientation of the tracking frame relative to the patient body 1 result from the imaging geometry.

In one particular implementation of the image-based tracking, the live images are (two-dimensional) x-ray fluoroscopy images and for acquiring these images, the tracking unit 7 comprises an x-ray fluoroscopy device. Moreover, in order to improve the visibility of the patient's vascular system, a suitable contrast agent may be injected in to the patient's blood vessels. This corresponds to the procedure applied in conventional angiography. Thus, in other words, the tracking unit 7 may acquire convention x-ray (fluoroscopy) angiography images.

As said above, when an image-based tracking technique is applied, the acquired live image may be displayed to the operator of the imaging system at the display unit 5 in order to visualize the patient's vascular system and the catheter device arranged therein. By viewing the images, the operator can directly identify the catheter device 2 and the US probe 3 in the live images and, thus, track the catheter device 2 and the US probe 3 with respect to the vascular system also shown in the images.

In order to improve the tracking of the US probe 3, it may be provided with a marker having an improved visibility in the images acquired using the tracking device. Further, the images acquired using the tracking unit 7 may be automatically evaluated in order to determine the position of the US probe 3 and make this position available for a further automatic processing as will be explained herein below.

In addition or as an alternative to the image-based tracking, the tracking unit 7 may track the US probe 3 with respect to a tracking frame by means of a non-image-based tracking technique. Examples of corresponding techniques that may be used in this embodiment include EM tracking, impedance tracking, optical shape sensing and satellite-based tracking. Using the non-image-based tracking technique, the tracking unit 7 can determine the position of the US probe 3 in a certain fixed tracking frame. The determined position is then already available for a further automatic processing thereof.

In order to visualize the position of the US probe 3 with respect to the patient's vascular system in case not live tracking images are acquired, the determined position may be indicated in a model of the patient's vascular system, where the model may be placed in the tracking frame in order to generate the aforementioned visualization, which in this case may include the model and a marker indicating the position of the US probe 3. The model may particularly be generated on the basis of an image of the patient's vascular system, which may e.g. be determined prior to the examination carried out using the imaging system. In particular, the model may be created on the basis of a CT angiography image as will be explained in more detail herein below.

Alternatively to the model-based visualization of the vascular tree, the position of the US probe 3 as determined by means of non-image-based tracking may be marked within live fluoroscopy images or other live images acquired by the tracking unit 7.

Moreover, in case of an automated motorized pullback procedure, the US probe 3 may be automatically tracked along the pullback path, which is usually known. In this implementation, the position of the US probe 3 on the path may parameterized on the basis of the distance covered by the US probe 3 when starting the pullback procedure at a certain start position, which may also be known. The distance may be calculated based on the velocity of the US probe 3 during the pullback procedure. The tracking frame corresponds to the pullback path in this implementation.

In addition to the aforementioned components, the imaging system comprises or cooperates with a CT device 8 configured to acquire a three-dimensional image of the vascular system of the patient 1 comprising the catheter device 2 and the US probe 3 included therein. In order to ease identification of the vascular tree in the images, a contrast agent may be in injected into the relevant region of the vascular system when the image is acquired. The image is acquired with respect to a certain imaging frame of reference and this imaging frame has a known relative position and orientation relative to the tracking frame described above. Therefore, it is possible to identify locations in the imaging frame and in the tracking frame that correspond to each other and it is possible to transfer objects from the imaging frame into the tracking frame.

Upon receipt of the CT image, the image is automatically evaluated in an evaluation unit 9 in order to determine values of one or more vessel parameters for blood vessels of the patient's vascular system. Such evaluation is also known as CT angiography (CTA) and therefore the related image is also referred to as CTA image herein. On the basis of the determined values, the evaluation unit 9 particularly identifies potential lesions in the patient's vascular tree as will be described in more detail herein below. These lesions may be indicated in the visualizations of the patient's vascular system generated by the tracking unit 7 as described above. In addition or as an alternative, the catheter may be automatically steered on the basis of the location of the potential lesion in the vascular system, e.g. during a motorized pullback procedure. The steering may particularly be performed such that the location of the potential lesion can be inspected especially carefully in the US images acquired by means of the US probe 3 and the US unit 4.

Further, in addition to or as an alternative to the aforementioned tracking techniques for tracking the US probe 3, the US probe 3 may be tracked along its path through the vascular tree on the basis of the US images acquired by means of the US probe 3 and on the basis of the CT image of the vascular tree. In the US images, certain landmark features may be identified and the same feature may be identified in the CT image in order to determine the position of the US probe 3 in the vascular tree when it acquires an image including a landmark feature. Examples of such landmark features include a plaque volume or a narrowing, for example, which are configured such that they allow for a unique identification of a position in the vascular tree. As will be further explained herein below, this tracking of the US probe 3 is particularly useful in case one or more image acquisition parameters of the US probe 3 are adapted based on the position of the US probe 3. Particularly in this embodiment, an additional tracking of the US probe 3 may be dispensed with.

Turning back to evaluation of the CTA image in the evaluation unit 9, a first step carried out in the evaluation unit 9 may comprise a segmentation of the vascular system shown in the CTA images to create a model of the patient's vascular system. In this process, the evaluation unit 9 determines a representation or, in another word, a model of the patient's vascular system. The segmentation may be performed in accordance with any suitable segmentation approach known to the person skilled in the art. On related approach includes vesselness filtering, which identifies tubular-like structures in the CTA images and segments the vascular tree on the basis of these structures. A further related approach uses region growing in which a location within a relevant structure, such as the inner or the wall of a blood vessel, is identified manually and automatically and in which further voxels of the structure are then determined on the basis of a predefined homogeneity criterion. An exemplary segmentation method that may be used in order to segment the patient's vascular tree is described in U. Jandt et al., "Automatic generation of 3D coronary artery centerlines using rotational X-ray angiography", Med Image Anal. 2009 Dec, 13(6):846-58, doi: 10.1016/j.media.2009.07.010.

Upon segmentation of the patient's vascular system in the CTA image, the segmented vascular tree may optionally be transformed into the tracking frame in order to create the aforementioned model-based visualization for tracking the catheter device 2 using the segmented vascular tree as the underlying model.

Further, the evaluation unit 9 may determine values for one or more parameters, which allow for identifying potential lesions. One related exemplary parameter is the wall thickness of the blood vessels of the coronary system. In particular, a larger wall thickness may be indicative of plaque. Blood vessels with a smaller wall thickness inhibit an increased risk of rupture. Therefore, the evaluation unit 9 may estimate the wall thickness for at least some of the blood vessels included in the segmented vascular tree. In so doing, estimates of the wall thickness may be estimated for the regular vessels in a regular predefined distance, for example. The estimated values may then be compared with a predefined upper and/or lower threshold value in order to identify the locations with abnormal wall thickness values, i.e. locations where the estimated value is larger than the upper threshold or smaller than the lower threshold. These locations correspond to positions in the vascular tree with a potential lesion. Namely, the locations may have an increased risk of rupture (in case the wall thickness is smaller than the lower threshold) or plaque deposition (in case the wall thickness is larger than the upper threshold).

Moreover, the evaluation unit 9 may be configured to detect locations with a calcified vessel wall and may determine a parameter indicating a calcification of the vessel wall. Calcified locations may generally also have an increased rupture risk and should therefore be further examined. In CT images, calcified regions can be identified on the basis of associated CT numbers or Hounsfield unit (HU) values. In order to determine calcifications of the vessel wall, the evaluation unit 9 may therefore evaluate the HU values in the CT image to determine regions in the areas of the vessel wall, which may contain calcifications. On the basis of this evaluation, the evaluation unit 9 may determine the parameter indicating a calcification of the vessel wall. This may be a binary parameter indicating whether or not a calcification has been detected and the case of a detected calcification may correspond to an abnormal value of the parameter. In a further variant, the parameter may indicate the amount of calcification and the evaluation unit 9 may further compare this parameter with a predetermined threshold value to detect an abnormal value of the parameter if it is greater than the threshold.

A further exemplary vessel parameter, which may be estimated in the evaluation unit 9 in addition or as an alternative, is the wall shear stress. This parameter quantifies the force per unit area created when a tangential force resulting from the blood flow acts on the inner surface of the blood vessel, i.e. on the endothelium. Studies have shown that wall shear stress can serve as an indicator for lesion growth and arterial remodeling. So, it has been shown that regions with an abnormal (e.g. unusually high or low) wall shear stress exhibit an increased risk of atherosclerosis-susceptibility, plaque growth and instability and of thrombosis. Unusually low values of the wall shear stress particularly indicate positions that are prone to a fast reduction of the vessel lumen, e.g. due to plaque growth. Unusually high values of the wall shear stress indicate positions have an increased risk of arterial remodeling and plaque rupture.

The evaluation unit 9 may estimate the wall shear stress (also referred to as endothelial shear stress in the art) for at least some of the blood vessels included in the segmented vascular tree. For this purpose, the evaluation unit 9 may perform a fluid dynamics simulation of the blood flow in the relevant blood vessels on the basis of the geometry of these blood vessels derived from the segmented vascular tree. In order to perform the fluid dynamics simulation, certain predefined assumption may be used with respect to the blood velocity and other relevant parameters of the blood flow. On the basis of these assumptions, the blood flow may be modeled using the Navier-Stokes equations, which may be solved for individual elements of the relevant blood vessels using a finite element method in a manner known to the person skilled in the art as such. On the basis of the simulated blood flow, the evaluation unit 9 may then estimate values of the wall shear stress for locations in the relevant blood vessels, e.g. in a predefined regular distance.

A related exemplary procedure, which may be applied by the evaluation unit 9, is disclosed in H. Hetterich et. al, "Coronary computed tomography angiography based assessment of endothelial shear stress and its association with atherosclerotic plaque distribution in-vivo", PLoS ONE 10(1): e0115408. doi:10.1371/journal.pone.0115408. However, it is likewise possible to apply any other suitable algorithm for calculating the wall shear tress known to the person skilled in the art.

Upon having estimated values of the wall shear stress as explained above, the evaluation unit 9 may compare these values with a predefined upper and/or lower threshold value in order to identify locations with abnormal wall shear stress values, which correspond to locations of potential lesions. In particular, the evaluation unit may identify locations where the wall shear stress is smaller than the lower threshold or larger than the upper threshold (i.e. locations with an abnormally high wall shear stress). Using appropriately selected threshold values, the locations identified in this manner correspond to the locations with an unusually low or high wall shear stress to which the aforementioned risk factors apply.

On the basis of the aforementioned results of the CTA evaluation, i.e. particularly on the basis of the identified locations associated with an abnormal parameter value of the wall shear stress and/or the wall thickness, a mapping unit 10 may modify the visualization for tracking the catheter device 2. In particular, the mapping unit 10 may modify the visualization such that identified locations are highlighted in the visualization.

For this purpose, the mapping unit 10 may transform the locations of the segmented vascular system associated with abnormal parameter values from the imaging frame into the tracking frame to identify the corresponding locations in the tracking frame. Then, mapping unit 10 may visually highlight these locations in the visualization of the vascular system generated with respect to the tracking frame, e.g. by coloring these locations using a pre-defined color and/or by superimpose arrows pointing to these locations on the visualization. This means that the locations with abnormal parameter values are highlighted in the x-ray fluoroscopy image forming the aforementioned visualizations, or in the model-based visualization of vascular system.

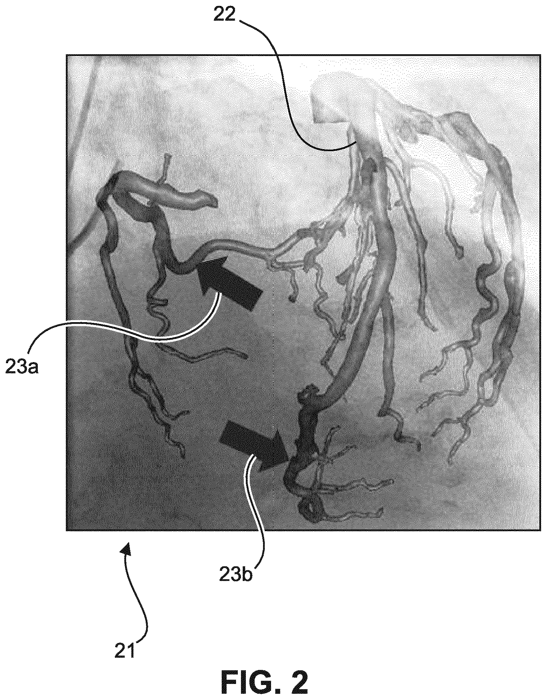

In a variation of this embodiment, the mapping unit 10 may overlay the live tracking image with the segmented vascular tree and highlight the locations associated with abnormal parameter values in the resulting visualization. In this variation, the mapping unit 10 may annotate the segmented vascular tree by providing the locations associated with abnormal parameters with markings, such a certain coloring or an arrow as explained above. Then, the mapping unit 10 may transform the annotated segmented vascular tree into the tracking frame and overlay the live tracking image with the transformed version of the annotated segmented vascular tree to generate a visualization output by means of the display unit 5. A corresponding visualization is schematically and exemplarily illustrated in Fig. 2, which shows a live image 21 of the vascular tree, which is overlaid by a segmented vascular tree 22. Locations in the vascular tree associated with abnormal values of a vessel parameter are marked by means of arrows 23a, 23b.

If a model-based visualization is used as explained above, the mapping unit 9 may likewise mark the locations associated with abnormal parameter values in the segmented vascular tree evaluated in the imaging frame as and may transform the segmented vascular tree including the markings into the tracking frame. The relevant locations may again be marked using a certain coloring and/or arrows. On the basis of the (transformed) markings, the mapping unit 10 may then create the highlighting for the relevant locations.

When highlighting the locations having abnormal values of a parameter, the mapping unit 10 may distinguish between locations where the value of the parameter is smaller than the lower threshold and locations where the value of the parameter is larger than the upper threshold (if such differentiation can be made - this is particularly the case with respect to wall shear stress and wall thickness). This may be achieved by highlighting the locations where the value of the parameter is smaller than the lower threshold using another color than it is used for highlighting locations where the value of the parameter is larger than the upper threshold.

Moreover, when abnormal values of two or more vessel parameters are determined in the evaluation unit 9, the mapping unit 10 may highlight the locations associated with abnormal values of all of these parameters but may highlight locations associated with abnormal values of each of the parameters in another way than locations associated with abnormal values of the other parameter(s). For instance, this may be achieved highlighting locations associated with abnormal values of one parameter using a different color than it is used for highlighting locations associated with abnormal values of the other parameter(s).

In a further embodiment, the mapping unit 10 not only highlights the locations associated with abnormal parameter values in the visualization of the vascular tree. Rather, the determined parameter values may be indicated for all locations of the blood vessels examined in the CTA evaluation. This may particularly be done with respect to parameters having more than two values, such as wall shear stress and wall thickness. In case of binary parameters, such as a parameter indicating a detected calcification, this is not necessary.

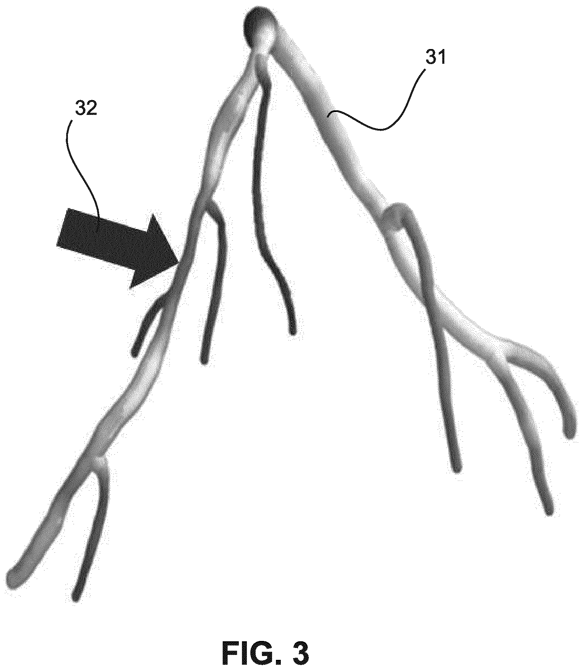

In order to indicate the determined parameter values for all examined locations, the evaluation unit 9 may quantize the determined parameter values in accordance with predefined quantization levels and may annotate the segmented vascular tree with the quantized values, e.g. by coloring the segmented vascular tree using one color for each quantization level. One quantization level may comprise the parameter values that are smaller than the lower threshold for the parameter and another quantization level comprises the values that are greater than the upper threshold for the parameter so that it is ensured that the locations associated with abnormal parameter values are highlighted as such. Thus, the locations associated with abnormal parameter values are indicated in the annotated segmented vascular tree. In addition, these locations may be additionally highlighted by further means, e.g. using arrows. A correspondingly annotated segmented vascular tree 31 is schematically and exemplarily illustrated in Fig. 3. As shown in this figure, regions of the vascular tree 31 are colored in accordance with quantized parameter values determined for locations in these regions. Moreover, a location associated with an abnormal parameter values is additionally marked by means of an arrow 32.

The mapping unit 10 may transform the annotated segmented vascular tree into the tracking frame and may overlay the live tracking image with the transformed annotated segmented vascular tree, e.g. by using a semi-transparent version thereof, in case of an image-based tracking. In case of a non image-based tracking, the mapping unit 10 may overlay the annotated segmented vascular tree over the model of the vascular tree used for tracking the catheter device 2 or - if this model corresponds to the segmented vascular tree - the tracking unit7 may generate the model-based visualization on the basis of the annotated version of the segmented vascular tree.

If values of two or more vessel parameters are determined in the evaluation unit 9, an annotated version of the segmented vascular system may be created with respect to each of the parameters in the aforementioned way. Further, the mapping unit 10 may create the visualization for tracking the catheter device using one selected annotated version of the vascular system at a time. The selection of the version may be made manually by the operator of the imaging system. Thus, the operator can select the parameter for which values are indicated in the visualization. Alternatively, the selection may be made automatically and the mapping unit may change the used version in regular intervals, for example.

In the aforementioned embodiments, values of vessel parameters - particularly abnormal values - are indicated in the visualization of the vascular tree used by the operator to track the catheter device 2 and the included US probe 3. On the basis of these indications, the operator can manually steer the US probe 3 to locations of potential lesions in order to inspect these locations for the purpose of examining these locations and/or of planning an interventional procedure.

In further embodiments, the catheter device 2 may be automatically steered on the basis of the parameter values determined in the evaluation unit 9. In particular, the catheter device 2 may be automatically steered on the basis of the abnormal parameter values such that the locations associated with the abnormal parameter values are imaged with greater depth of detail.

In one related implementation, the steering unit 6 may control an automated motorized pullback procedure on the basis of the information about the locations associated with abnormal parameter values. In this implementation, the pullback may be carried out with a first predefined velocity of the catheter device 2 and the included US probe 3 and the velocity may be reduced to a second predefined value when the US probe 3 passes a location associated with an abnormal parameter value. Hereby, it is possible to image these locations with a greater depth of detail.

In order for the steering unit 6 to perform such a procedure, it is provided with information about the locations associated with abnormal parameter values and with information for determining the phases of the pullback procedure when the US probe 3 passes these locations. In one realization, the control unit 7 may rely on an automatic tracking of the US probe 3 as described above in order to determine the relevant phases of the pullback procedure. Thus, the steering unit 6 is provided with information about the locations associated with abnormal parameter values which refer to the tracking frame and it is provided with the positions of the US probe 3 as determined by means of the tracking unit 7. During the pullback procedure, the steering unit 6 compares the position of the US probe 3 with the locations associated with abnormal parameter values and reduces the pullback velocity when the distance between the position of the US probe 3 and one of these locations is smaller than a predetermined distance value.

In an alternative realization, the steering unit 6 may determine the distance that US probe 3 has covered during the pullback procedure on the basis of the velocity of the US probe during the procedure and may be provided with distance corresponding to locations associated with abnormal parameter values.

In this realization, the pullback starts at a known position of the US probe 3, which may e.g. be manually indicated by the operator of the imaging system. In order to indicate the start position, the operator may mark the initial position of the US probe 3 in the visualization used for tracking the catheter device, for example. Then, the path of the US probe 3 through the vascular tree during the pullback procedure may be determined, e.g. on the basis of the segmented vascular tree. This may be done in the mapping unit 10. Upon having determined the path, the mapping unit 10 may determine lengths of path segments from the start position to the locations associated with abnormal parameter values along the determined path, and these lengths may be provided to the steering unit 6.

During the pullback procedure, the steering unit 6 compares the distance covered by the US probe 3 as determined on the basis of the probe velocity with the received lengths and determines that the US probe 3 passes a location associated with an abnormal parameter value, when the distance covered by the US probe 3 corresponds to one of the received lengths. In this case, the steering unit 6 reduces the velocity of the US probe 3 in order to facilitate the acquisition of US images with a greater detail depth. The reduction of the velocity may particularly allow for imaging the relevant location with a higher frame rate. The frame rate may be kept constant during the pullback procedure so that a reduction of the velocity allows for acquiring more images per unit length, which corresponds to an increased image resolution along the pullback direction of the acquisition. In a variant of this embodiment, the location associated with an abnormal parameter value may correspond to a landmark feature that can be identified in both the CT image and in the US images acquired using the US probe 3, or the location may be adjacent to such a landmark feature preceding the location on the pullback path. In this case, the US images may be evaluated (essentially) in real time and the steering unit 6 may reduce the velocity of the US probe 3 if the landmark prove is detected in one or more acquired US images. If the location associated with an abnormal parameter value can be identified in this way, an additional tracking of the US probe 3 may be dispensed with.

In a further embodiment, the steering units 6 adapts one or more image acquisition parameters of the US probe 3, when the US probe 3 passes a location associated with an abnormal value of the vessel parameter. This allows for imaging this location using an adapted image acquisition setting that differs from the image acquisition setting used for imaging other regions of the vascular tree. The image acquisition setting may be selected such that an examination of the potential lesion at the identified location is facilitated.

In this embodiment, the steering unit 6 may compare the position of the US probe 3 with the identified locations associated with abnormal parameter values while the US probe 3 moves through the vascular tree. The movement may be steered manually or automatically, e.g. as explained above. The position of the US probe 3 may again be determined on the basis of a live fluoroscopy image, on the basis of a non image-based tracking method or on the basis of the US images and the CT image as explained above.

When the steering unit 6 determines that the distance between the US probe 3 and an identified location is smaller than a predetermined threshold value and/or that a landmark feature corresponding to the identified location or located adjacent to the identified location is included in one or more images acquired by means of the US probe 3, the steering unit may adapt the image acquisition parameter to change the image acquisition setting of the US probe 3. When the US probe 3 has passed the location and the steering unit 6 has determined that the distance between the US probe 3 and the location is again larger than the threshold value, the steering unit 3 may reverse the adaption of the image acquisition parameter so that the image acquisition settings return to the settings used before passing the location. Further, the image acquisition parameter may be adapted on the basis of the vessel parameter having an abnormal value at a certain location. Thus, the steering unit 6 may determine on the basis of the vessel parameter which image acquisition parameter is adapted and how the adaption is carried out. Since the vessel parameter is indicative of the type of potential lesion at the location, this allows for adapting the image acquisition setting of the US probe to the specific potential lesion.

The image acquisition parameters that may be adapted by the steering unit 6 may include the US frequency applied by the US probe 3 for imaging and/or the frame rate used by the US probe to acquire images. For instance, if the vessel parameter associated with an abnormal value indicates a calcification of the vessel wall at a certain location, the US frequency may be reduced when imaging this location since a lower US frequency allows for a better visualization of the calcified vessel wall. In a further, example the frame rate may be reduced at locations with an increased thickness of the vessel wall in order to increase the imaging depth. Moreover, the US probe 3 may be operable in an imaging mode and in a flow meter mode for measuring and visualizing blood flow, and the steering unit 6 may control the US probe 3 to switch to the flow meter mode at the location associated with abnormal parameter value. This allows for examining the blood flow at this location, which may provide further insight into the severity of the potential lesion and may facilitate the decision about its treatment.

Further, it is possible to apply special image evaluation procedures for evaluating the US images acquired at a location associated with an abnormal value of a vessel parameter in the US unit 5 or in another device. One example of such an evaluation procedure comprises a virtual histology evaluation that is based a spectrum analysis of IVUS-derived radiofrequency data. A exemplary virtual histology evaluation procedure, we may be applied in the US unit 5, is described in A. Konig, V. Klauss, "Virtual histology", Heart, 2007 Aug, 93(8): 977-982, doi: 10.1136/hrt.2007.116384.

The predetermined image evaluation procedure may be selected for evaluating images that are acquired when the distance between the position of the US probe and the at least one location is smaller than a predetermined threshold. In order to achieve this, the steering unit 6 may compare the position of the US probe 3 with the identified locations associated with abnormal parameter values while the US probe 3 moves through the vascular tree as explained above. When the steering unit 6 determines that the distance between the US probe 3 and an identified location is smaller than a predetermined threshold value, it may mark the acquired images such that they are provided to the predetermined image evaluation procedure.

In the embodiments described above, guidance for a manual or automatic steering of an intravascular US catheter device 1 is provided on the basis of vessel parameter values determined on the basis of a diagnostic CT image by means of a CT angiography evaluation. However, the invention is not limited to an angiographic evaluation of CT images. Rather, diagnostic images acquired using another imaging modality, such as MR imaging, may be analyzed in order to determine vessel parameters. Moreover, the invention is not limited to three-dimensional diagnostic images. Likewise, it is also possible to acquire two-dimensional diagnostic images in order to determine certain vessel parameters. For instance, an abnormal narrowing of a vessel may also be detected in two-dimensional diagnostic x-ray images.

Further, values of the wall shear stress, of the wall thickness and of a parameter indicative of calcifications have been referred to above as examples of vessel parameter values on the basis of which the intravascular US catheter device 2 may be steered. However, it is likewise possible to provide guidance for steering the intravascular US catheter device 2 on the basis of values of other vessel parameters, particularly on the basis of abnormal values of such vessel parameters. Related examples include the plaque burden, which parameterizes a ratio between the total vessel volume and the lumen volume, and blood flow parameters, which may indicate the blood velocity and/or the type of blood flow (e.g. laminar or turbulent).

Variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

## Claims

1. A system for guiding an intravascular ultrasound catheter device (2) comprising an ultrasound probe (3) to a potential lesion in a vascular tree, comprising:
a localization unit (6; 7) configured to provide position information of the ultrasound probe (3) for displaying to a user in a visualization of the vascular tree and/or for an automated processing of the position information;
an evaluation unit (9) configured to
receive a diagnostic image of the vascular tree (22; 31),
determine values of at least one vessel parameter for a plurality of locations in the vascular tree (22; 31) on the basis of the diagnostic image, and
identify at least one location in the vascular tree (22; 31) associated with an abnormal value of the at least one vessel parameter; and
a mapping unit (10) configured to provide a visual indication (23a; 23b; 32) of the at least one location in the visualization of the vascular tree (22; 31) and/or to provide information about the at least one location for use in the automated processing of the position information.

2. The system as defined in claim 1, wherein the at least one vessel parameter comprises at least one of a wall shear stress acting on an inner wall of vessels of the vascular tree (22; 31), a wall thickness of the walls of vessels of the vascular tree (22; 31) and a parameter indicating a calcification of the vessel wall.

3. The system as defined in claim 1, wherein the evaluation unit (9) is configured to identify an abnormal value of the at least one parameter if the value of the parameter is smaller than a predetermined lower threshold or greater than a predetermined upper threshold.

4. The system as defined in claim 1, wherein
the localization unit (6; 7) is configured to provide the position information of the ultrasound probe with respect to a tracking frame,
the evaluation unit (9) is configured to identify the at least one location in an imaging frame of the diagnostic image, and
the mapping unit (10) is configured to transform the at least one location into the tracking frame to provide the visual indication (23a; 23b, 32) of the least one location in the visualization of the vascular tree (22; 31) and/or to provide the information about the at least one location for use in the automatic processing of the position information.

5. The system as defined in claim 1, wherein the localization unit (7) is configured to acquire live images of the vascular tree (22), the live images corresponding to the visualization of the vascular tree.

6. The system as defined in claim 5, wherein the mapping unit (10) is configured to provide a visual indication (23a; 23b) of the at least one location in the live images.

7. The system as defined in claim 1, wherein the evaluation unit (9) is configured to segment the vascular tree in the diagnostic image to obtain a segmented vascular tree (21; 31) and to determine the values of the at least one vessel parameter on the basis of the segmented vascular tree (21; 31).

8. The system as defined in claims 5 and 7, wherein the mapping unit (10) is configured to annotate the segmented vascular tree with an indication of the at least one location to obtain an annotated segmented vascular tree (21; 31) and to overlay the live images with the annotated segmented vascular tree (21; 31).

9. The system as defined in claim 1, wherein the localization unit (6; 7) is configured to automatically track the position of the ultrasound probe (3) to provide position information for the automated processing.

10. The system as defined in claim 1, further comprising a steering unit (6) configured to automatically steer the intravascular ultrasound catheter device (2), wherein the automated processing of the position information comprises an automated steering of the intravascular ultrasound catheter device (2) based on the position information by means of the steering unit (6).

11. The system as defined in claim 10, wherein the steering unit (6) is configured to steer the intravascular ultrasound catheter device (2) in an automated pullback procedure on the basis of information about the at least on location associated with the abnormal value of the vessel parameter.

12. The system as defined in claim 11, wherein the steering unit (7) is configured to compare a position of the ultrasound probe (3) during the automated pullback procedure with the at least one location and to control a velocity of the ultrasound probe (3) during the automated pullback procedure on the basis of the result of the comparison.

13. The system as defined in claim 1, wherein the automated processing of the position information comprises an adaption of at least one image acquisition parameter of the ultrasound probe (3) and/or to select a predetermined image evaluation procedure for evaluating an image acquired by the ultrasound probe (3) on the basis of a distance between a position of the ultrasound probe (3) and the at least one location.

14. A method for guiding an intravascular ultrasound catheter device (2) comprising an ultrasound probe (3) to a potential lesion in a vascular tree, comprising:
providing position information of the ultrasound probe (3) for displaying to a user in a visualization of the vascular tree and/or for an automated processing of the position information;
receiving a diagnostic image of the vascular tree (21; 31);
determining values of at least one vessel parameter for a plurality of locations in the vascular tree (21; 31) on the basis of the diagnostic image;
identifying at least one location in the vascular tree associated with an abnormal value of the vessel parameter; and
providing a visual indication (23a; 23b; 32) of the at least one location in the visualization of the vascular tree (21; 31) and/or providing information about the at least one location for use in the automated processing of the position information.

15. A computer program comprising program code for causing a computer device to carry out a method as defined in claim 14, when the computer program is executed in the computer device.
